# EUROPEAN PATENT SPECIFICATION

(11) **EP 3 210 577 B1**
(45) Date of publication and mention of the grant of the patent: **18.04.2018**
(21) Application number: 16157557.6
(22) Date of filing: 26.02.2016
(51) Int. Cl.: A61F 2/44

(54) **BONE FUSION DEVICE**
KNOCHENVERBINDUNGSVORRICHTUNG
DISPOSITIF DE FUSION OSSEUSE

(43) Date of publication of application: 30.08.2017
(73) Proprietor: OSSAWARE Biotech Co., Ltd., Changhua County 509 (TW)
(72) Inventor: HUANG, Max, 509 Changhua County (TW)
(74) Representative: Viering, Jentschura & Partner mbB Patent- und Rechtsanwälte

(56) References cited:
- EP-A1- 2 764 851
- US-A1- 2015 057 753

## Description

### BACKGROUND OF THE INVENTION

### 1. Field of the Invention

The present invention relates to a bone fusion device, and more particularly to a bone fusion device able to generate a micro motion to help bones grow and to provide a stable engagement.

### 2. Description of the Prior Art

Normally, a bone fusion surgery is used for solving the degeneration of the spinal bones or facet joints due to agedness or other external causes.

As disclosed in Taiwan Patent application number 104208837 titled "spinal fusion device", 103220076 titled "implantable artificial intervertebral structure", 100119603 titled "spinal implant and device thereof", 101202457 titled "cervical vertebra fusion device", and so on, the teeth on the top and bottom surfaces of a main body provide an acting force for propping two bones (the vertebra or the joints) to a predetermined distance.

Wolff's law states that bone in a healthy person or animal will adapt to the loads under which it is placed. If loading on a particular bone increases, the bone will remodel itself over time to become stronger to resist that sort of loading. The devices of the aforesaid patents only provide a single acting force applied to two bones. When the human body is moved, the retaining effect of the device is not stable. More unfortunately, it is unable to bring obvious bone-conducted stimulation, so the efficiency to grow bones needs to be improved.

Accordingly, the inventor of the present invention has devoted himself based on his many years of practical experiences to solve these problems.

EP 2 764 851 A1 discloses a staged expansion vertebra retaining device comprises a main body and a screw inserted into the main body. The distal end of the screw is provided with a push block. One end of the main body has at least two movable support units. Each movable support unit is split by a U-shaped groove to form at least two movable support surfaces which are spaced and arranged in the form of inner and outer layers. The movable support surface is first expanded by the push block, and then the other movable support surface is continuously expanded at an appropriate angle.

US 2015/057753 A1 discloses an expandable spinal implant configured for positioning within a space between adjacent vertebral bodies includes an upper body, a lower body, and first and second pins. The lower body is adapted to slidably receive the upper body. Grooves are disposed on opposing side surfaces of the upper body, and grooves are disposed on an inner surface of a bore defined through the lower body. Corresponding first and second apertures are defined through an end surface of the lower body thereby permitting advancement of the first and second pins within the first and second apertures, grooves of the lower body, and a corresponding pair of grooves of the upper body to secure the position of the upper body relative to the lower body.

### SUMMARY OF THE INVENTION

The primary object of the present invention is to provide a bone fusion device. The bone fusion device comprises a main body and a movable frame. The main body has a front end and an opposing rear end, a top surface and an opposing bottom surface, and an accommodation hole longitudinally penetrating the top surface and the bottom surface. The top surface and the bottom surface are provided with a plurality of first teeth. The first teeth are spaced and disposed between the front end and the rear end. The movable frame is located in the accommodation hole. The movable frame has a first end and an opposing second end at front and rear ends thereof, a first surface and a second surface at upper and lower ends thereof, and a through hole longitudinally penetrating the first surface and the second surface. The first end of the movable frame is fixed to the front end of the main body. The first surface and the second surface are provided with a plurality of second teeth. The second teeth are spaced and disposed between the first end and the second end. The second end of the movable frame is transversely formed with a groove to define an upper portion and a lower portion. The upper portion and the lower portion can be elastically expanded or retracted with the first end as a fulcrum. When the main body is implanted between two bones, the first teeth provide a first acting force, the second teeth provide a second acting force, and the second acting force is not equal to the first acting force.

Accordingly, the second acting force of the second teeth to act on the two bones and the first acting force of the first teeth to act on the two bones are differential and complementary to strengthen the stability of engagement. The first teeth and the second teeth can slightly stimulate the two bones to expedite fusion of the two bones so as to improve the shortcomings of the prior art.

### BRIEF DESCRIPTION OF THE DRAWINGS

FIG. 1 is a perspective view of the present invention;
FIG. 2 is an exploded view of the present invention;
FIG. 3 is a sectional view of the present invention;
FIG. 4 is a schematic view of the present invention implanted between two bones;
FIG. 5 is a schematic view of a first embodiment of the present invention implanted between two bones;
FIG. 6 is a schematic view of a second embodiment of the present invention implanted between two bones;
FIG. 7 is a schematic view of a first embodiment of the movable frame fixed to the main body of the present invention;
FIG. 8 is a schematic view of a second embodiment of the movable frame fixed to the main body of the present invention;
FIG. 9 and FIG. 10 are schematic views of a third embodiment of the movable frame fixed to the main body of the present invention;
FIG. 11 is a schematic view of a fourth embodiment of the movable frame fixed to the main body of the present invention;
FIG. 12 is a schematic view of another embodiment of the movable frame of the present invention;
FIG. 13 is a schematic view of the main body and the movable frame implanted between two bones of FIG. 12;
FIG. 14 is a schematic view of a further embodiment of the movable frame of the present invention; and
FIG. 15 is a yet schematic view of a further yet embodiment of the movable frame of the present invention.

### DETAILED DESCRIPTION OF THE PREFERRED EMBODIMENTS

Embodiments of the present invention will now be described, by way of example only, with reference to the accompanying drawings.

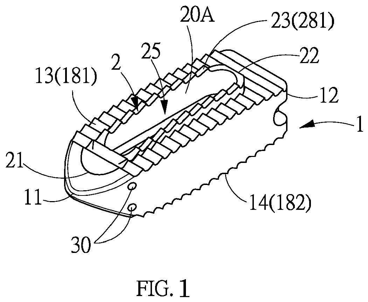

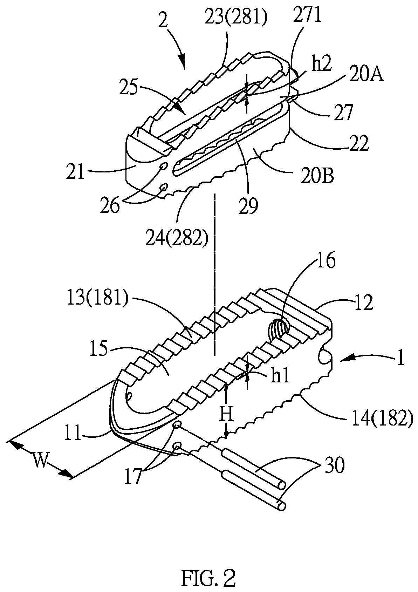

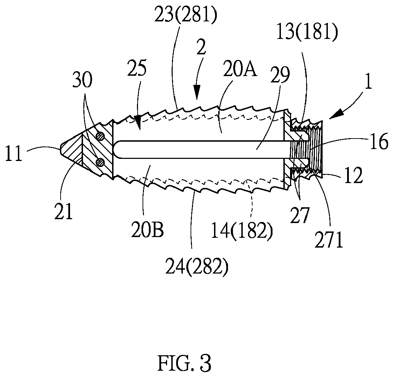

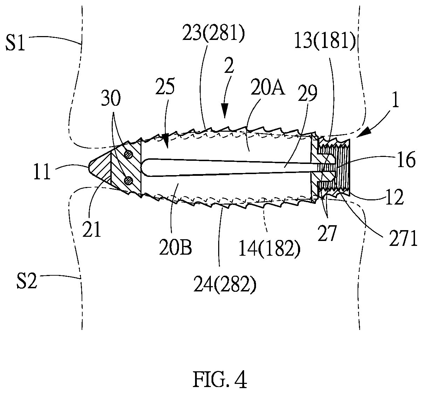

As shown in FIG. 1 through FIG. 4, the present invention comprises a main body 1 and a movable frame 2.

The main body 1 has a front end 11 and an opposing rear end 12. The man body 1 further has a raised curved top surface 13 and a raised curved bottom surface 14. The curvatures of the top surface 13 and the bottom surface 14 are close to the curvatures of two adjacent bones S1, S2 (referring to the spines or joints). A height defined by the top surface 13 and the bottom surface 14 is gradually reduced toward the front end 11 and a width defined by left and right sides of the main body 1 is gradually reduced toward the front end 11, enabling the front end 11 to be easily implanted between the two bones S1, S2. The main body 1 further has an accommodation hole 15 which longitudinally penetrates the top surface 13 and the bottom surface 14 and has a closed side wall. The rear end 12 of the main body 1 is transversely formed with a mounting hole 16 in communication with the accommodation hole 15. The mounting hole 16 has inner threads for screwed engagement of a tool member T. The main body 1 is formed with two first holes 17 which are disposed close to the front end 11 and penetrates the left and right sides of the main body 1. The first holes 17 are in communication with the accommodation hole 15. The top surface 13 and the bottom surface 14 have a plurality of first teeth 181, 182 which are evenly disposed between the front end 11 and the rear end 12. The first teeth 181, 182 are arranged at an equal interval between the front end 11 and the rear end 12. The first teeth 181, 182 are in the form of ratchets and have a tooth height h1 which is gradually reduced toward the front end 11, such that the first teeth 181, 182 cooperate with the front end 11 of the main body 1 to be implanted between the two bones S1, S2 in a one-way manner, providing a first acting force to be implanted between the two bones S1, S2.

The movable frame 2 is inserted into the accommodation hole 15, and has a first end 21 and an opposing second end 22 at front and rear ends thereof. Upper and lower ends of the movable frame 2 have raised curved first and second surfaces 23, 24. The curvatures of the first and second surface 23, 24 are close to the curvatures of the two adjacent bones S1, S2. The movable frame 2 further has a through hole 25 which longitudinally penetrates the first and second surfaces 23, 24 and has a closed side wall. The first end 21 of the movable frame 2 corresponds to the front end 11 of the main body 1, and the second end 22 is suspended relative to the rear end 12 of the main body 1. The first end 21 is formed with second holes 26 corresponding to the first holes 17 for insertion of coupling members 30. The first end 21 is fixed to the front end 11 of the main body 1. The second end 22 is provided with a push post 27 which concentrically extends into the mounting hole 16. An outer wall of the push post 27 has a push surface 271. The first surface 23 and the second surface 24 have a plurality of second teeth 281, 282 which are evenly disposed between the first end 21 and the second end 22. The second teeth 281, 282 are arranged at an equal interval between the first end 21 and the second end 22. The second teeth 281, 282 are in the form of ratchets and have a tooth height h2 which is gradually reduced toward the first end 21. The second teeth 281, 282 and first teeth 181, 182 are disposed in a staggered arrangement, such that the first teeth 181, 182 and the second teeth 281, 282 cooperate with the front end 11 of the main body 1 to be implanted between the two bones S1, S2 in a one-way manner. The movable frame 2 is transversely cut in half from an outer end of the push post 27 to form a groove 29 which doesn't penetrate through the first end 21 of the movable frame 2, such that the movable frame 2 is symmetrically formed with an upper portion 20A and a lower portion 20B. The upper portion 20A and the lower portion 20B can be elastically expanded with the first end 21 as the fulcrum, enabling the second teeth 281, 282 of the first surface 23 and the second surface 24 to provide a second acting force to the two bones S1, S2. The second acting force is not equal to the first acting force to generate a differential complementation, which reinforces the engagement of the main body 1 and the movable frame 2 when the human body is moved. What is important is that the first teeth 181, 182 or the second teeth 281, 282 can slightly friction and stimulate the two bones S1, S2 (bone-conducted stimulation) to expedite fusion of the two bones S1, S2.

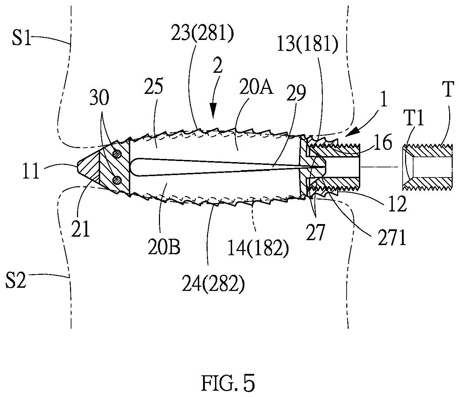

Referring to FIG. 1 through FIG. 5, before the present invention is implanted between the two bones S1, S2, the tool member T having a conical hole T1 is screwed to the mounting hole 16. The conical hole T1 of the tool member T is screwedly moved to act on the push surface 271 of the push post 27 for the push post 27 to link the upper portion 20A and the lower portion 20B of the movable frame 2 to retract toward the groove 29, such that the second teeth 281, 282 can be implanted between the two bones S1, S2 smoothly. After implantation, the tool member T is taken out to release the outward expanding elasticity of the second teeth 281, 282.

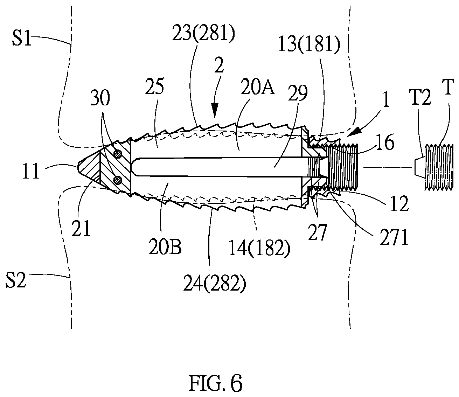

Referring to FIG. 1, FIG. 2, FIG. 3, FIG. 4, FIG. 6, the second teeth 281, 282 of the movable frame 2 of the present invention is kept within the accommodation hole 15, not extending out of the first teeth 181, 182 for the present invention to be implanted between the two bones S1, S2 as the aforesaid patents. The tool member T having a conical post T2 is screwed to the mounting hole 16. The conical post T2 of the tool member T is screwedly inserted into the groove 29 of the push post 27, enabling the conical post T2 to control the degree of outward expansion of the upper portion 20A and the lower portion 20B of the movable frame 2 and applying a second acting force to be implanted between the two bones S1, S2. The second acting force is controlled to be different from the first acting force so as to differentially stimulate the growth of the two bones S1, S2.

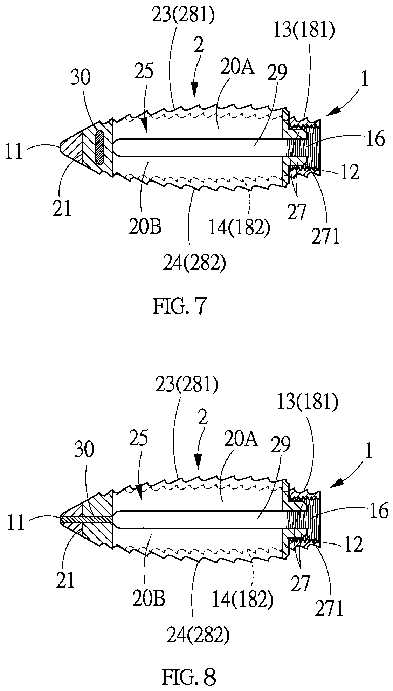

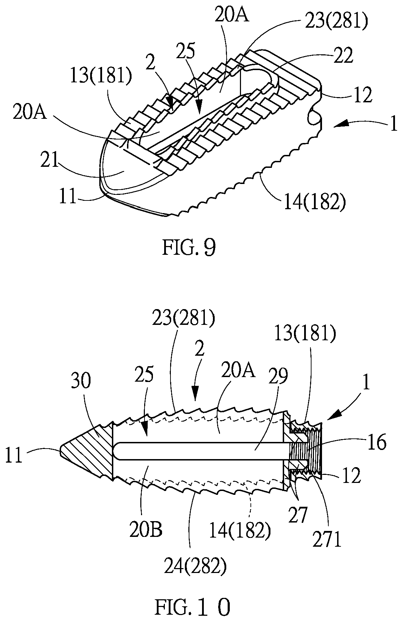

As shown in FIG. 1 and FIG. 7, the shape and the number of the first holes 17, the second holes 26, and the coupling members 30 can be changed. As shown in FIG. 8, the direction of the first holes 17, the second holes 26, and the coupling members 30 can be also changed. Specifically, the first end 21 of the movable frame 2 can be fixedly connected to the accommodation hole 15 relative to the position of the front end 11 of the main body 1. This belongs to the present invention. Furthermore, as shown in FIG. 9 and FIG. 10, the movable frame 2 can be integrally formed in the accommodation hole 15 of the main body 1 if the manufacturing technique and the cost are allowed.

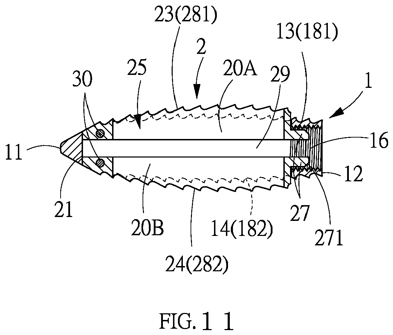

Referring to FIG. 11, the upper portion 20A and the lower portion 20B of the movable frame 2 are separately mounted in the accommodation hole 15 through different coupling members 30, so that the groove 29 is configured to separate the upper portion 20A and the lower portion 20B of the movable frame 2. This also belongs to the present invention.

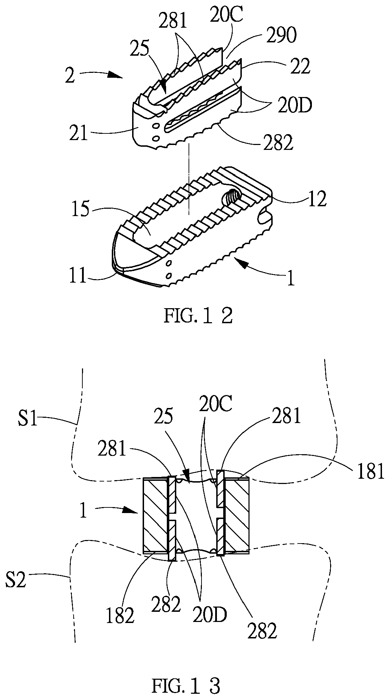

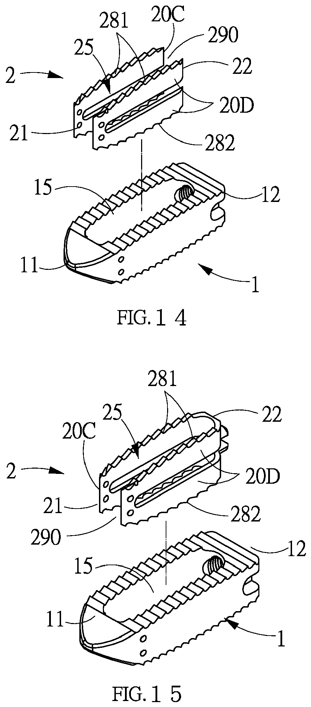

As shown in FIG. 12, the movable frame 2 is located in the accommodation hole 15. The first end 21 is fixedly connected to the front end 11 of the main body 1, and the second end 22 is suspended relative to the rear end 12 of the main body 1. The movable frame 2 is vertically longitudinally cut in half to form an opening 290 which penetrates the second end 22 but doesn't penetrate the first end 21. The opening 290 is in communication with the through hole 25. The movable frame 2 is symmetrically formed with a left portion 20C and a right portion 20D. The second teeth 281, 282 of the left portion 20C and the right portion 20D can be expanded upward or downward with the first end 21 as the fulcrum. As shown in FIG. 13, when the present invention is implanted between the two bones S1, S2, the second teeth 281, 282 of the left portion C and the second teeth 281, 282 of the right portion D form a differential motion corresponding to the different gaps formed by the two bones S1, S2. When the human body is moved, the second teeth 281, 282 can slightly friction and stimulate the two bones S1, S2 (bone-conducted stimulation) to expedite fusion of the two bones S1, S2. In addition, as shown in FIG. 14, the opening 290 vertically longitudinally penetrates the first end 21 and the second end 22. The left portion 20C and the right portion 20D are separately fixed to the front end 11 of the main body 1. This embodiment enhances the differential motion effect of the second teeth 281, 282 of the left portion 20C and the right portion 20D. As shown in FIG. 15, the movable frame 2 is vertically longitudinally cut in half to form an opening 290 which penetrates the first end 21 but doesn't penetrate the second end 22. The opening 290 is in communication with the through hole 25. The movable frame 2 is symmetrically formed with a left portion 20C and a right portion 20D. This embodiment can slow down the differential motion effect of the second teeth 281, 282 of the left portion 20C and the right portion 20D of FIG. 13. The embodiment of FIG. 15 is equivalent to that of FIG. 1.

Furthermore, the main body 1 and the movable frame 2 can adopt a metallic or high-polymer material. The metallic material is selected from one of titanium, tantalum, cobalt, stainless steel, liquid metal, and nickel. The high-polymer material is selected from one of PEEK, PEKK, PU, PE, carbon fiber, PVC, PC, and PP.

To sum up, the whole arrangement of the present invention is novel. The second acting force of the second teeth to act on the two bones and the first acting force of the first teeth to act on the two bones are differential and complementary to strengthen the stability of engagement. The present invention can slightly stimulate the two bones to expedite fusion of the two bones.

### Reference Number

- S1, S2: adjacent bones
- T: tool member
- T1: conical hole
- 1: main body
- 11: front end
- 12: opposing rear end
- 13: raised curved top surface
- 14: raised curved bottom surface
- H: height
- W: width
- 15: accommodation hole
- 16: mounting hole
- 17: first hole
- 181, 182: first teeth
- h1: tooth height
- 2: movable frame
- 20A: upper portion
- 20B: lower portion
- 20C: left portion
- 20D: right portion
- 21: first end
- 22: opposing second end
- 23: first surface
- 24: second surface
- 25: through hole
- 26: second hole
- 27: push post
- 271: push surface
- 281, 282: second teeth
- h2: tooth height
- 29: groove
- 290: opening
- 30: coupling member

## Claims

1. A bone fusion device, comprising:
a main body (1), the main body (1) having a front end (11) and an opposing rear end (12), a top surface (13) and an opposing bottom surface (14), and an accommodation hole (15) longitudinally penetrating the top surface (13) and the bottom surface (14); the top surface (13) and the bottom surface (14) being provided with a plurality of first teeth (181, 182), the first teeth (181, 182) being spaced and disposed between the front end (11) and the rear end (12); and
a movable frame (2) located in the accommodation hole (15), the movable frame (2) having a first end (21) and an opposing second end (22) at front and rear ends thereof, a first surface (23) and a second surface (24) at upper and lower ends thereof, and a through hole (25) longitudinally penetrating the first surface (23) and the second surface (24); the first end (21) of the movable frame (2) being fixed to the front end (11) of the main body (1), the first surface (23) and the second surface (24) being provided with a plurality of second teeth (281, 282), the second teeth (281, 282) being spaced and disposed between the first end (21) and the second end (22); the second end (22) of the movable frame (2) being transversely formed with a groove (29) to define an upper portion (20A) and a lower portion (20B) for the upper portion (20A) and the lower portion (20B) to be elastically expanded or retracted with the first end (21) as a fulcrum, wherein when the main body (1) is implanted between two bones (S1, S2), the first teeth (181, 182) provide a first acting force, the second teeth (281, 282) provide a second acting force, and the second acting force is not equal to the first acting force.

2. The bone fusion device as claimed in claim 1, wherein the movable frame (2) is vertically longitudinally cut in half to form an opening (290) penetrating the second end (22), the opening (290) is in communication with the through hole (25), enabling the movable frame (2) to form a left portion (20C) and a right portion (20D), and the second teeth (281, 282) of the left portion (20C) and the right portion (20D) can be elastically expanded with the first end (21) as the fulcrum.

3. The bone fusion device as claimed in claim 1, wherein the movable frame (2) is vertically longitudinally cut in half to form an opening (290) penetrating the first end (21), and the opening (290) is in communication with the through hole (25), enabling the movable frame (2) to form a left portion (20C) and a right portion (20D).

4. The bone fusion device as claimed in claim 1, 2 or 3, wherein the rear end (12) of the main body (1) is transversely formed with a mounting hole (16) in communication with the accommodation hole (15), the mounting hole (16) has inner threads for screwed engagement of a tool member (T); the second end (22) of the movable frame (2) is provided with a push post (27) which concentrically extends into a front edge of the mounting hole (16), and the groove (29) horizontally penetrates the push post (27) in half.

5. The bone fusion device as claimed in claim 4, wherein an outer wall of the push post (27) has a push surface (271), a tool member (T) has a conical hole (T1) corresponding to the push surface (271), the conical hole (T1) of the tool member (T) is screwedly moved to act on the push surface (271) of the push post (27) for the push post (27) to link the upper portion (20A) and the lower portion (20B) of the movable frame (2) to retract toward the groove (29), such that the second teeth (281, 282) can be implanted between the two bones (S1, S2), and the tool member (T) is taken out to release the outward expanding elasticity of the second teeth (281, 282) after implantation.

6. The bone fusion device as claimed in claim 4, wherein a tool member (T) has a conical post (T2), the second teeth (281, 282) of the movable frame (2) is kept within the accommodation hole (15), after the main body (1) is adapted to be implanted between the two bones (S1, S2), the conical post (T2) is screwedly moved to be inserted into the groove (29) of the push post (27) to link the upper portion (20A) and the lower portion (20B) of the movable frame (2) to expand or retract for applying a second acting force to the two bones (S1, S2).

7. The bone fusion device as claimed in claim 1, 2, or 3, wherein the front end (11) of the main body (1) is formed with a first hole (17), the first hole (17) is in communication with the accommodation hole (15), and the first end (21) of the movable frame (2) is formed with a second hole (26) corresponding to the first hole (17) for insertion of a coupling member (30), such that the first end (21) of the movable frame (2) is fixed to the front end (11) of the main body (1).

8. The bone fusion device as claimed in claim 1, 2, or 3, wherein the first end (21) of the movable frame (2) is integrally formed with the front end (11) of the main body (1).

9. The bone fusion device as claimed in claim 1, 2, or 3, wherein the groove (29) is arranged half from the second end (22) of the movable frame (2) toward the first end (21) and adapted to selectively penetrate or don't penetrate the first end (21) of the movable frame (2).

10. The bone fusion device as claimed in claim 1, 2, or 3, wherein the top surface (13) and the bottom face (14) of the man body (1) are raised and curved, the top surface (13) and the bottom surface (14) have curvatures close to the curvatures of the two bones (S1, S2), a height defined by the top surface (13) and the bottom surface (14) is gradually reduced toward the front end (11), a width defined by left and right sides of the main body (1) is gradually reduced toward the front end (11), the first surface (23) and the second surface (24) of the movable frame (2) are raised and curved, the first surface (23) and the second surface (24) have curvatures close to the curvatures of the two bones (S1, S2), the first teeth (181, 182) and the second teeth (281, 282) are in the form of ratchets and have a tooth height (h1, h2) which is gradually reduced toward the first end (11) of the main body (1), such that the first teeth (181, 182) and the second teeth (281, 282) cooperate with the front end (11) of the main body (1) adapted to be implanted between the two bones (S1, S2) in a one-way manner.

## Patentansprüche

1. Ein Knochenverschmelzungsapparat, umfassend:
einen Grundkörper (1); der Grundkörper (1) eine vordere Endfläche (11) und auf der anderen Seite eine hintere Endfläche (12), eine obere Fläche (13) und auf der anderen Seite eine untere Fläche (14) aufweist, wobei eine Aufnahmeöffnung (15) der Länge nach durch die obere Fläche (13) und durch die untere Fläche (14) verläuft; die obere Fläche (13) und die untere Fläche (14) mit mehreren ersten Zähnen (181, 182) gebildet sind; die ersten Zähne (181, 182) in einem Abstand zueinander und zwischen der vorderen Endfläche (11) und der hinteren Endfläche (12) gebildet sind; und
einen Verschieberahmen (2), der in der Aufnahmeöffnung (15) angeordnet ist; der Verschieberahmen (2) eine erste Endfläche (21) und auf der anderen Seite eine zweite Endfläche (22) an dessen vorderen bzw. hinteren Endflächen aufweist; eine erste Oberfläche (23) und eine zweite Oberfläche (24) an dessen oberen bzw. unteren Endteilen aufweist; ein Durchgangsloch (25) der Länge durch die erste Oberfläche (23) und durch die zweite Oberfläche (24) gebildet ist; die erste Endfläche (21) des Verschieberahmens (2) an der vorderen Endfläche (11) des Grundkörpers (1) vorgesehen ist; die erste Oberfläche (23) und die zweite Oberfläche (24) mehrere zweite Zähne (281, 282) aufweist; die zweiten Zähne (281, 282) in einem Abstand zueinander und zwischen der ersten Endfläche (21) und der zweiten Endfläche (22) vorgesehen sind; auf der zweiten Endfläche (22) des Verschieberahmens (2) quer eine Nut (29) verläuft, um einen oberen Teil (20A) und einen unteren Teil (20B) zu bilden, damit dieser obere Teil (20A) und der untere Teil (20B) elastisch ausgestreckt oder eingezogen werden und dabei die erste Endfläche (21) als einen Drehpunkt dient; beim Implantieren des Grundkörpers (1) zwischen zwei Knochen (S1, S2) mit den ersten Zähnen (181, 182) eine erste wirkende Kraft erzeugt wird; mit den zweiten Zähnen (281, 282) eine zweite wirkende Kraft erzeugt wird, wobei die zweite wirkende Kraft sich von der ersten wirkenden Kraft unterscheidet.

2. Der Knochenverschmelzungsapparat nach Anspruch 1, worin der Verschieberahmen (2) vertikal und der Länge nach in zwei Hälften unterteilt ist, um eine Öffnung (290) zu bilden, die durch die zweite Endfläche (22) gebildet ist; die Öffnung (290) mit dem Durchgangsloch (25) verbunden ist; so dass mit dem Verschieberahmen (2) einen linken Teil (20C) und einen rechten Teil (20D) gebildet werden; die zweiten Zähne (281, 282) des linken Teils (20C) und des rechten Teils (20D) elastisch ausgestreckt werden können, wobei die erste Endfläche (21) als Drehpunkt dient.

3. Der Knochenverschmelzungsapparat nach Anspruch 1, worin der Verschieberahmen (2) vertikal und der Länge nach in zwei Hälften unterteilt ist, um eine Öffnung (290) zu bilden, die durch die erste Endfläche (21) gebildet ist, wobei die Öffnung (290) mit dem Durchgangsloch (25) verbunden ist, so dass mit dem Verschieberahmen (2) einen linken Teil (20C) und einen rechten Teil (20D) gebildet werden.

4. Der Knochenverschmelzungsapparat nach Anspruch 1, 2 oder 3, worin auf der hinteren Endfläche (12) des Grundkörpers (1) quer ein Montageloch (16), der mit der Aufnahmeöffnung (15) verbunden ist, gebildet ist; das Montageloch (16) ein Innengewinde aufweist, um einen Werkzeugteil (T) in dieses einzuschrauben; die zweite Endfläche (22) des Verschieberahmens (2) mit einer Schubstange (27), die sich konzentrisch in eine vordere Kante des Montagelochs (16) erstreckt, gebildet ist, wobei die Nut (29) horizontal in der Hälfte der Schubstange (27) verläuft.

5. Der Knochenverschmelzungsapparat nach Anspruch 4, worin eine Außenwand der Schubstange (27) eine Schubfläche (271) aufweist; der Werkzeugteil (T) eine konische Bohrung (T1) in Übereinstimmung mit der Schubfläche (271) aufweist; die konische Bohrung (T1) des Werkzeugteils (T) im geschraubten Zustand bewegt wird, um auf die Schubfläche (271) der Schubstange (27) zu wirken, damit die Schubstange (27) den oberen Teil (20A) mit dem unteren Teil (20B) des Verschieberahmens (2) verbindet, um auf die Nut (29) eingezogen zu werden, so dass die zweiten Zähne (281, 282) zwischen die zwei Knochen (S1, S2) eingesetzt werden können; der Werkzeugteil (T) herausgenommen wird, um die sich nach außen erstreckende Elastizität der zweiten Zähne (281, 282) nach dem Implantieren auszulösen.

6. Der Knochenverschmelzungsapparat nach Anspruch 4, worin der Werkzeugteil (T) einen konischen Stift (T2) aufweist; die zweiten Zähne (281, 282) des Verschieberahmens (2) in der Aufnahmeöffnung (15) behalten werden; nach dem Anpassen des Grundkörpers (1) zum Implantieren zwischen zwei Knochen (S1, S2) der konische Stift (T2) im geschraubten Zustand zum Einsetzen in die Nut (29) der Schubstange (27) bewegt wird, um den oberen Teil (20A) mit dem unteren Teil (20B) des Verschieberahmens (2) zu verbinden und auszustrecken oder einzuziehen, um eine zweite wirkende Kraft auf die zwei Knochen (S1, S2) auszuüben.

7. Der Knochenverschmelzungsapparat nach Anspruch 1, 2 oder 3, worin die vordere Endfläche (11) des Grundkörpers (1) eine erste Öffnung (17) aufweist; die erste Öffnung (17) mit der Aufnahmeöffnung (15) verbunden ist; die erste Endfläche (21) des Verschieberahmens (2) eine zweite Öffnung (26) in Übereinstimmung mit der ersten Öffnung (17) aufweist, um ein Kupplungsglied (30) einzusetzen, so dass die erste Endfläche (21) des Verschieberahmens (2) an die vordere Endfläche (11) des Grundkörpers (1) befestigt wird.

8. Der Knochenverschmelzungsapparat nach Anspruch 1, 2 oder 3, worin die erste Endfläche (21) des Verschieberahmens (2) einstückig mit der vorderen Endfläche (11) des Grundkörpers (1) gebildet ist.

9. Der Knochenverschmelzungsapparat nach Anspruch 1, 2 oder 3, worin die Nut (29) auf halbem Weg von der zweiten Endfläche (22) des Verschieberahmens (2) auf die erste Endfläche (21) zu angeordnet ist und wahlweise durch die erste Endfläche (21) des Verschieberahmens (2) verläuft oder auch nicht durch diese verläuft.

10. Der Knochenverschmelzungsapparat nach Anspruch 1, 2 oder 3, worin die obere Fläche (13) und die untere Fläche (14) des Grundkörpers (1) erhoben und gebogen sind; die obere Fläche (13) und die untere Fläche (14) Krümmungen nahe zu den Krümmungen der zwei Knochen (S1, S2) aufweisen; eine mit der oberen Fläche (13) und der unteren Fläche (14) gebildete Höhe auf die vordere Endfläche (11) zu allmählich verringert ist; eine mit den linken und rechten Seiten des Grundkörpers (1) gebildete Dicke auf die vordere Endfläche (11) zu allmählich verringert ist; die erste Oberfläche (23) und die zweite Oberfläche (24) des Verschieberahmens (2) erhoben und gebogen sind; die erste Oberfläche (23) und die zweite Oberfläche (24) Krümmungen nahe zu den Krümmungen der zwei Knochen (S1, S2) aufweisen; die ersten Zähne (181, 182) und die zweiten Zähne (281, 282) in Form von Ratschen gebildet sind und eine Zahnhöhe (h1, h2) aufweisen, die auf die erste Endfläche (11) des Grundkörpers (1) zu allmählich verringert ist, so dass die ersten Zähne (181, 182) und die zweiten Zähne (281, 282) mit der vorderen Endfläche (11) des zwischen die zwei Knochen (S1, S2) einseitig zu implantierenden Grundkörpers (1) zusammenwirken.

## Revendications

1. Dispositif de fusion osseuse, **caractérisé par le fait qu'**il comprend :
un corps principal (1), le corps principal (1) comportant une extrémité avant (11) et une extrémité arrière disposée à l'opposé (12), une surface supérieure (13) et une surface inférieure disposée à l'opposé (14), et un trou de logement (15) pénétrant de manière longitudinale la surface supérieure (13) et la surface inférieure (14) ; la surface supérieure (13) et la surface inférieure (14) comportant une pluralité de premières dents (181, 182), les premières dents (181, 182) étant espacées et disposées entre l'extrémité avant (11) et l'extrémité arrière (12) ; et
une armature amovible (2) située dans le trou de logement (15), l'armature amovible (2) comportant une première extrémité (21) et une seconde extrémité disposée à l'opposé (22) sur ses extrémités avant et arrière, une première surface (23) et une seconde surface (24) sur ses extrémités supérieure et inférieure, et un trou traversant (25) pénétrant de manière longitudinale la première surface (23) et la seconde surface (24) ; la première extrémité (21) de l'armature amovible (2) étant fixée à l'extrémité avant (11) du corps principal (1), la première surface (23) et la seconde surface (24) comportant une pluralité de secondes dents (281, 282), les secondes dents (281, 282) étant espacées et disposées entre la première extrémité (21) et la seconde extrémité (22) ; la seconde extrémité (22) de l'armature amovible (2) comportant de manière transversale une rainure (29) pour définir une partie supérieure (20A) et une partie inférieure (20B) de façon à permettre à la partie supérieure (20A) et à la partie inférieure (20B) d'être étendues ou rétractées de manière élastique avec la première extrémité (21) comme point d'appui, **caractérisé par le fait que** lorsque le corps principal (1) est implanté entre deux os (S1, S2), les premières dents (181, 182) fournissent une première force agissante, les secondes dents (281, 282) fournissent une seconde force agissante, et la seconde force agissante n'est pas égale à la première force agissante.

2. Dispositif de fusion osseuse selon la revendication 1, **caractérisé par le fait que** l'armature amovible (2) est verticalement et de manière longitudinale coupée en deux pour former une ouverture (290) pénétrant la seconde extrémité (22), l'ouverture (290) est en communication avec le trou traversant (25), permettant à l'armature amovible (2) de former une partie gauche (20C) et une partie droite (20D), et les secondes dents (281, 282) de la partie gauche (20C) et de la partie droite (20D) peuvent être étendues de manière élastique avec la première extrémité (21) comme point d'appui.

3. Dispositif de fusion osseuse selon la revendication 1, **caractérisé par le fait que** l'armature amovible (2) est verticalement et de manière longitudinale coupée en deux pour former une ouverture (290) pénétrant la première extrémité (21), et l'ouverture (290) est en communication avec le trou traversant (25), permettant à l'armature amovible (2) de former une partie gauche (20C) et une partie droite (20D).

4. Dispositif de fusion osseuse selon la revendication 1, 2 ou 3, **caractérisé par le fait que** l'extrémité arrière (12) du corps principal (1) comportant de manière transversale un trou d'assemblage (16) en communication avec le trou de logement (15), le trou d'assemblage (16) comporte un filetage interne pour l'engagement à vis d'un élément d'outil (T) ; la seconde extrémité (22) de l'armature amovible (2) comporte une tige de poussée (27) qui s'étend concentriquement dans un rebord avant du trou d'assemblage (16), et la rainure (29) pénètre de manière horizontale la tige de poussée (27) de moitié.

5. Dispositif de fusion osseuse selon la revendication 4, **caractérisé par le fait qu'**une paroi externe de la tige de poussée (27) comporte une surface de poussée (271), l'élément d'outil (T) comporte un trou conique (T1) correspondant à la surface de poussée (271), le trou conique (T1) de l'élément d'outil (T) est déplacé par vissage pour agir sur la surface de poussée (271) de la tige de poussée (27) pour permettre à la tige de poussée (27) de relier la partie supérieure (20A) et la partie inférieure (20B) de l'armature amovible (2) pour se rétracter en direction de la rainure (29), de sorte que les secondes dents (281, 282) peuvent être implantées entre les deux os (S1, S2), et l'élément d'outil (T) est retiré pour libérer l'élasticité s'étendant en direction de l'extérieur des secondes dents (281, 282) après implantation.

6. Dispositif de fusion osseuse selon la revendication 4, **caractérisé par le fait que** l'élément d'outil (T) comporte une tige conique (T2), les secondes dents (281, 282) de l'armature amovible (2) sont conservées dans le trou de logement (15), après le corps principal (1) est adapté pour être implanté entre les deux os (S1, S2), la tige conique (T2) est déplacée par vissage pour être insérée dans la rainure (29) de la tige de poussée (27) pour relier la partie supérieure (20A) et la partie inférieure (20B) de l'armature amovible (2) pour s'étendre ou se rétracter pour appliquer une seconde force agissante sur les deux os (S1, S2).

7. Dispositif de fusion osseuse selon l'une quelconque des revendications 1, 2 ou 3, **caractérisé par le fait que** l'extrémité avant (11) du corps principal (1) comporte un premier trou (17), le premier trou (17) est en communication avec le trou de logement (15), et la première extrémité (21) de l'armature amovible (2) comporte un second trou (26) correspondant au premier trou (17) pour l'insertion d'un élément de liaison (30), de sorte que la première extrémité (21) de l'armature amovible (2) est fixée à l'extrémité avant (11) du corps principal (1).

8. Dispositif de fusion osseuse selon l'une quelconque des revendications 1, 2 ou 3, **caractérisé par le fait que** la première extrémité (21) de l'armature amovible (2) est formée intégralement avec l'extrémité avant (11) du corps principal (1).

9. Dispositif de fusion osseuse selon l'une quelconque des revendications 1, 2 ou 3, **caractérisé par le fait que** la rainure (29) est disposée de moitié depuis la seconde extrémité (22) de l'armature amovible (2) en direction de la première extrémité (21) et pénètre ou ne pénètre pas de manière sélective la première extrémité (21) de l'armature amovible (2).

10. Dispositif de fusion osseuse selon l'une quelconque des revendications 1, 2 ou 3, **caractérisé par le fait que** la surface supérieure (13) et la surface inférieure (14) du corps principal (1) sont élevées et courbées, la surface supérieure (13) et la surface inférieure (14) présentent des parties courbées près des parties courbées des deux os (S1, S2), une hauteur définie par la surface supérieure (13) et la surface inférieure (14) est graduellement réduite en direction de l'extrémité avant (11), une largeur définie par les côtés gauche et droit du corps principal (1) est graduellement réduite en direction de l'extrémité avant (11), la première surface (23) et la seconde surface (24) de l'armature amovible (2) sont élevées et courbées, la première surface (23) et la seconde surface (24) présentent des parties courbées près des parties courbées des deux os (S1, S2), les premières dents (181, 182) et les secondes dents (281, 282) ont la forme de cliquets et présentent une hauteur de dent (h1, h2) qui est graduellement réduite en direction de la première extrémité (11) du corps principal (1), de sorte que les premières dents (181, 182) et les secondes dents (281, 282) coopèrent avec l'extrémité avant (11) du corps principal (1) pour être implantées entre les deux os (S1, S2) d'une manière unidirectionnelle.
